# FASCICULE DE BREVET EUROPEEN

(11) **EP 1 184 656 B1**
(45) Date de publication et mention de la délivrance du brevet: **28.01.2015**
(21) Numéro de dépôt: 01402233.9
(22) Date de dépôt: 27.08.2001
(51) Int. Cl.: C23F 1/26, C25F 3/00, G01N 1/32, G01N 33/20

(54) **Procédé de révélation structurale pour superalliages monocristallins**
Verfahren für die strukturelle Entwicklung von monokristallinen Superlegierungen
Method for structural development of monocrystalline superalloys

(30) Priorité: 28.08.2000 FR 0010979
(43) Date de publication de la demande: 06.03.2002
(73) Titulaire: SNECMA, 75015 Paris (FR)
(72) Inventeur: Ruimi, Michel, 75019 Paris (FR); Poutonnet, Sylvie, 77000 Vaux le Penil (FR); Poubanne, Philippe, 91240 Vigneux sur Seine (FR)
(74) Mandataire: Joly, Jean-Jacques

(56) Documents cités:
- EP-A- 0 971 041
- WO-A-93/04370
- WO-A-98/20341
- US-A- 4 445 988
- US-A- 4 548 903

## Description

### Arrière-plan de l'invention

Il est bien connu d'utiliser des alliages métalliques réfractaires, en particulier des superalliages monocristallins, pour réaliser des pièces destinées à être exposées, en service, à des températures élevées. Il en est ainsi notamment pour des pièces de turbines à gaz, telles que des aubes fixes ou mobiles de turboréacteurs.

Les pièces en superalliage monocristallin telles que les aubes précitées sont fabriquées par fonderie avec contrôle du processus de solidification.

Une inspection des pièces venues de fonderie est nécessaire pour détecter d'éventuels défauts, tels qu'inclusions, fissures ou criques, qui sont susceptibles d'affecter leur tenue mécanique en service.

A cet effet, il est connu de procéder à une attaque macrographique de la surface des pièces au moyen d'un bain acide. Un bain couramment utilisé est un bain contenant des ions chlorure, en particulier un bain contenant de l'acide chlorhydrique et du perchlorure de fer en solution aqueuse.

La déposante a constaté que le traitement par ce bain perd de son efficacité avec certains types de superalliages, en particulier ceux à base de nickel contenant des éléments tels que le rhénium ou le ruthénium. Or, l'utilisation de ces types de superalliages peut s'avérer nécessaire pour autoriser des températures de service toujours plus élevées qui permettent d'accroître le rendement des turboréacteurs

Le document WO 93/04370 décrit un procédé de contrôle destructif par prélèvement d'échantillon dans un lingot ou une barre d'acier et attaque électrochimique avec un bain d'acide chlorhydrique pour éliminer une partie de surface et révéler des défauts internes.

Le document US 4 548 903 décrit un procédé comprenant une attaque chimique d'une surface police d'un échantition d'alliage métallique pour en révéler la microstructure tandis que le document US 4 44-5 988 décrit un procédé comprenant une attaque électrochimique d'une surface d'une pièces métallique pour détecter des signes de déformation plastique.

### Objet et resumé de l'invention

L'invention a pour but de proposer un procédé de revélation structurale qui permette de réaliser une inspection aisée et efficace de pièces en superalliages lorsque ceux-ci contiennent des éléments tels que le rhénium ou le ruthénium.

Ce but est atteint grâce à une tel que défini dans la revendication 1.

L'acide faible est par exemple choisi parmi l'acide acétique, l'acide tartrique et l'acide citrique.

L'association avec un acide faible permet de compenser l'effet sélectif de l'attaque par l'acide phosphorique vis-à-vis de certains constituants du superalliage, un acide faible tel que l'acide acétique ou autre ayant une activité sur d'autres constituants, en particulier le nickel, qui est un constituant de base habituel des superalliages.

Le bain peut comprendre en outre un constituant capable d'homogénéiser la vitesse de dissolution lors de l'attaque électrochimique, par exemple un alcool secondaire tel que du glycol.

Selon un aspect particulier de l'invention, l'étape préalable d'attaque macrographique est réalisée avantageusement par voie chimique au moyen d'un bain acide classique comprenant des ions chlorure et perchlorure de fer. La réalisation d'une telle attaque préalable sur des superalliages contenant notamment du rhénium et/ou du ruthénium provoque un noircissement de la pièce en raison de la formation persistante d'oxyde de rhénium et/ou de ruthénium en surface. L'attaque électrochimique réalisée ensuite conformément à l'invention permet un blanchiment ou nettoyage de la surface par élimination de ces oxydes. Une inspection visuelle de la pièce peut alors être réalisée aisément. La réalisation de ces deux attaques chimique et électrochimique, l'une après l'autre, permet de combiner les efficacités des deux bains utilisés vis-à-vis des différents éléments du superalliage.

L'utilisation de la voie électrochimique permet d'accroître la réactivité, notamment vis-à-vis d'alliages à base de nickel enrichis en éléments tels que Re et Ru. La pièce en superalliage est immergée dans le bain. Elle est disposée en phase anodique face à une cathode spécifique ayant une forme appropriée de façon à obtenir une distribution homogène des lignes de courant sur la pièce.

### Brève description des dessins

L'invention sera mieux comprise à la lecture de la description faite ci-après, à titre indicatif mais non limitatif, au cours de laquelle il sera fait référence aux dessins annexés sur lesquels :
- la figure 1 est une photographie montrant l'efficacité du procédé selon l'invention pour le blanchiment de la surface d'une pièce en superalliage monocristallin en vue de son inspection visuelle ;
- la figure 2 montre un exemple de réalisation d'un montage pour la mise en oeuvre d'un procédé selon l'invention ; et
- la figure 3 montre des aubes branchées en anodes sur le montage de la figure 2.

### Description détaillée de modes de réalisation de l'invention

L'invention est plus particulièrement applicable à la révélation structurale de pièces en superalliages monocristallins contenant du rhénium et/ou du ruthénium.

Des superalliages monocristallins de ce type à base de nickel, destinés plus particulièrement à la réalisation d'aubes de turboréacteurs, sont décrits notamment dans le document FR 98 08 693. Leur composition en masse est : 3,5 à 7,5 % de Cr, 0 à 1,5 % de Mo, 1,5 à 5,5 % de Re, 0 à 5,5 % de Ru, 3,5 à 8,5 % de W, 5 à 6,5 % d'AI, 0 à 2,5 % de Ti, 4,5 à 9 % de Ta, 0,08 à 0,12 % de Hf, 0,08 à 0,12 % de Si, le reste étant du nickel ou des impuretés éventuelles. Bien entendu, le domaine d'application de l'invention n'est pas limité à des alliages répondant à cette définition particulière.

Selon un mode particulier de mise en oeuvre de l'invention, un procédé de révélation structurale pour une pièce en superalliage monocristallin comprend les étapes qui consistent à :
- réaliser sur la pièce une première attaque macrographique chimique au moyen d'un premier bain acide comprenant, de façon classique, des ions chlorure,
- effectuer un rinçage de la pièce,
- sécher la pièce à l'air comprimé,
- réaliser sur la pièce une deuxième attaque par voie électrochimique au moyen d'un deuxième bain acide comprenant, conformément à l'invention, de l'acide phosphorique et au moins un acide faible,
- effectuer un rinçage de la pièce, et
- effectuer éventuellement un séchage de la pièce, celle-ci étant apte à être inspectée visuellement pour détecter d'éventuels défauts de fabrication.

La première attaque chimique peut être effectuée avec un bain contenant de l'acide chlorhydrique et du perchlorure de fer en solution dans de l'eau. Les proportions en masse d'acide chlorhydrique et de perchlorure de fer sont par exemple respectivement comprises entre 70 et 80 % et entre 5 et 10 %, le reste étant de l'eau.

L'attaque est réalisée par immersion de la pièce dans le bain à une température d'environ 50°C à 70°C pendant une durée comprise entre 15 min et 30 min. Bien entendu, une pluralité de pièces peuvent être traitées de façon concomitante.

Après rinçage de la pièce à l'eau déionisée, la deuxième attaque par voie électrochimique est effectuée avec le bain contenant de l'acide phosphorique et au moins un acide faible. Ce dernier est avantageusement choisi parmi l'acide acétique, l'acide tartrique et l'acide citrique. Un constituant supplémentaire peut être ajouté ayant une fonction d'adoucissement de la morsure chimique. On pourra utiliser à cet effet un alcool secondaire.

A titre d'exemple, les proportions en volume des différents constituants du bain peuvent être les suivantes : 30 à 80 % d'acide phosphorique, 3 à 15 % d'acide faible tel que de l'acide acétique, et 0 à 15 % d'alcool secondaire tel que du glycol, le reste étant de l'eau.

La pièce immergée dans le bain est reliée à une anode et est entourée par une cathode. La forme de la cathode est choisie en fonction de celle de la pièce pour assurer au mieux une distribution homogène des lignes de courant (équipotentielles) dans le bain et, par conséquent, sur la pièce elle-même de manière à traiter celle-ci de façon sensiblement uniforme. La densité de courant anodique est relativement élevée, par exemple comprise entre 10 à 60 A/dm². La durée de l'attaque électrochimique peut être relativement brève, par exemple comprise entre 1 min et 5 min.

### Exemple 1

Un barreau en superalliage monocristallin à base de nickel, du type défini dans le document précité FR 98 08 693, contenant du rhénium et du ruthénium a été traité de la façon suivante :
- première attaque macrographique chimique dans un bain acide contenant, en masse, 77 % d'acide chlorhydrique, 7 % de perchlorure de fer et 16 % d'eau à une température de 55°C pendant 20 min,
- rinçage à l'eau déionisée,
- séchage à l'air comprimé,
- deuxième attaque par voie électrochimique réalisée sur une partie du barreau immergée dans un bain acide contenant, en volume, environ 60 % d'acide phosphorique, environ 10 % d'acide acétique, environ 5 % de glycol, le reste étant de l'eau ; la partie immergée du barreau est branchée en phase anodique et est entourée par une cathode cylindrique en tôle d'acier inoxydable ; l'attaque chimique est réalisée pendant 2 min,
- rinçage à l'eau distillée, et
- séchage sous courant d'air chaud.

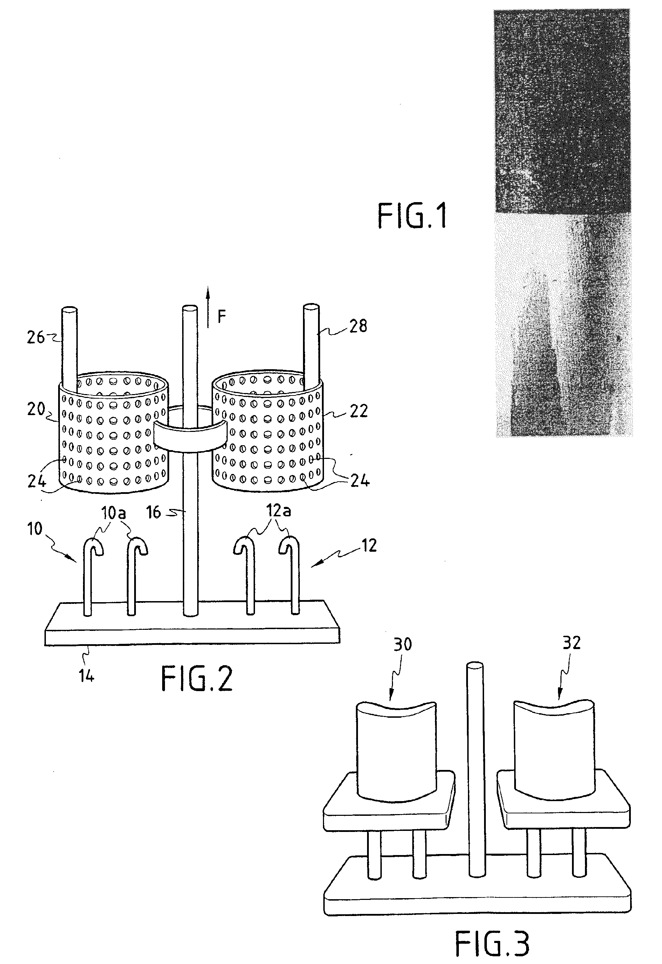

La figure 1 montre le barreau obtenu, dans sa partie supérieure, après la première attaque chimique et, dans sa partie inférieure, après la deuxième attaque électrochimique.

La surface de la partie supérieure est noircie par la présence d'oxydes de rhénium et de ruthénium, rendant l'inspection visuelle difficile, voire impossible.

La surface de la partie inférieure, nettoyée ("blanchie") par la deuxième attaque électrochimique, laisse parfaitement voir des défauts de fabrication éventuels (notamment, sur la photographie de la figure 1, la présence d'une inclusion de couleur plus sombre).

### Exemple 2

Des aubes, venues de fonderie, en superalliage monocristallin à base de nickel, du type défini dans le document précité FR 98 08 693 contenant 4 % de rhénium et 4 % de ruthénium, ont été traitées de la façon décrite dans l'exemple 1 à l'exception du fait qu'elles ont été intégralement soumises à la deuxième attaque électrochimique en étant complètement immergées dans le bain acide.

On a utilisé le montage de la figure 2 permettant le traitement simultané de deux aubes.

Ce montage, disposé dans un bac, comprend deux anodes 10, 12, montées sur un support commun 14 relié par un barreau 16 à une borne d'alimentation extérieure (non représentée), l'ensemble étant réalisé en acier inoxydable. Les anodes sont sous forme de tiges présentant à leur extrémité libre une partie recourbée 10a, 12a déformable élastiquement par pincement.

A chaque pièce est associée une cathode, respectivement 20, 22. Les cathodes ont une forme sensiblement cylindrique. Elles sont en acier inoxydable présentant une pluralité de trous 24 dans leur paroi. Les cathodes sont reliées l'une à l'autre et sont montées aux extrémités de deux barreaux 26, 28 également en acier inoxydable. Les barreaux 26, 28 sont reliés à une borne d'alimentation extérieure (non représentée).

Le barreau 16 peut coulisser (flèche F) de manière à amener les anodes dans une position où elles sont entourées par les cathodes.

La figure 3 montre partiellement le montage de la figure 2 avec deux aubes 30, 32 montées sur les anodes. Le support des aubes et le contact électrique avec celles-ci a été réalisé par engagement des anodes, et pincement de leurs extrémités recourbées, dans des canaux internes des aubes. Bien entendu, différentes formes de montages peuvent être utilisées en fonction des formes et possibilités offertes par les pièces à traiter.

Après montage des aubes, les cathodes ont été mises en place autour de celles-ci et l'ensemble a été immergé dans le bain acide. La dimension et la forme des cathodes étaient spécifiquement choisies pour qu'elles entourent les aubes en ménageant avec celles-ci un intervalle relativement constant, par exemple d'environ 30 mm. Une bonne répartition des lignes de courant dans le bain a pu ainsi être obtenue, permettant un traitement relativement uniforme de la surface des aubes.

Les trous 24 des cathodes favorisent le dégagement gazeux qui se produit (O₂) et limitent l'échauffement du bain par effet Joule en évitant un confinement de l'espace entre anode et cathode en regard.

L'attaque électrochimique a été réalisée dans les conditions de l'exemple 1, avec une densité anodique de courant égale à environ 30 A/dm². Après 2 min d'attaque électrochimique, les aubes ont été retirées du bain, rincées et séchées. Elles présentaient une surface extérieure claire laissant aisément voir d'éventuels défauts de fabrication.

Le montage décrit ci-avant permet de traiter simultanément deux pièces. Bien entendu, des montages similaires peuvent être utilisés pour le traitement d'une seule pièce ou, simultanément, d'un nombre de pièces supérieur à deux.

## Revendications

1. Procédé de révélation structurale pourl'inspection visuelle de surface de pièce fabriquée en superalliage monocristallin contenant du rhénium et/ou du ruthénium, le procédé comprenant une phase d'attaque macrographique chimique au moyen d'un premier bain acide, **caractérisé en ce que**, après la première étape, une étape subséquente de nettoyage de la surface de la pièce est réalisée par attaque électrochimique au moyen d'un deuxième bain différent du premier comprenant de l'acide phosphorique et au moins un acide faible,

2. Procédé selon la revendication 1, **caractérisé en ce que** l'acide faible est choisi parmi l'acide acétique, l'acide tartrique et l'acide citrique.

3. Procédé selon l'une quelconque des revendications 1 et 2, **caractérisé en ce que** l'on utilise un deuxième bain comprenant en outre un alcool secondaire.

4. Procédé selon la revendication 3, **caractérisé en ce que** l'alcool secondaire est le glycol.

5. Procédé selon l'une quelconque des revendications 1 à 4, **caractérisé en ce que** l'on utilise un deuxième bain comprenant, en volume, 30 à 80 % d'acide phosphorique, 3 à 15 % d'acide faible et 0 à 15 % d'alcool secondaire.

6. Procédé selon l'une quelconque des revendications 1 à 5, **caractérisé en ce que** la pièce est immergée dans le deuxième bain, branchée en anode et entourée d'une cathode.

7. Procédé selon la revendication 6, **caractérisé en ce que** l'on utilise une cathode ayant une forme adaptée à celle de la pièce afin de ménager avec celle-ci un intervalle sensiblement uniforme.

8. Procédé selon l'une quelconque des revendications 2 et 7, **caractérisé en ce que** l'on utilise une cathode percée de trous.

9. Procédé selon l'une quelconque des revendications 1 à 8, **caractérisé en ce que** l'on réalise l'étape d'attaque électrochimique pendant une durée comprise entre 1 min et 5 min.

10. Procédé selon l'une quelconque des revendications 1 à 9, **caractérisé en ce que** l'étape d'attaque macrographique chimique est réalisée par immersion dans un premier bain comprenant des ions chlorure et perchlorure de fer.

11. Procédé selon l'une quelconque des revendications 1 à 10 pour le contrôle visuel d'aubes de turboréacteurs en superalliage monocristallin contenant du rhénium et/ou du ruthénium,

## Patentansprüche

1. Verfahren zur Strukturaufdeckung für die Sichtprüfung einer Oberfläche eines Teils, das aus Rhenium und/oder Ruthenium enthaltender monokristalliner Superlegierung gefertigt ist, wobei das Verfahren eine Phase eines makrographischen chemischen Ätzens mittels eines ersten sauren Bades umfasst, **dadurch gekennzeichnet, dass** nach dem ersten Schritt ein nachfolgender Schritt zur Reinigung der Oberfläche des Teils durch elektrochemisches Ätzen mittels eines von dem ersten verschiedenen zweiten Bades, das Phosphorsäure und wenigstens eine schwache Säure umfasst, durchgeführt wird.

2. Verfahren nach Anspruch 1, **dadurch gekennzeichnet, dass** die schwache Säure aus Essigsäure, Weinsäure und Zitronensäure ausgewählt ist.

3. Verfahren nach einem der Ansprüche 1 und 2, **dadurch gekennzeichnet, dass** ein zweites Bad verwendet wird, das ferner einen sekundären Alkohol umfasst.

4. Verfahren nach Anspruch 3, **dadurch gekennzeichnet, dass** der sekundäre Alkohol Glykol ist.

5. Verfahren nach einem der Ansprüche 1 bis 4, **dadurch gekennzeichnet, dass** ein zweites Bad verwendet wird, das 30 bis 80 Vol.-% Phosphorsäure, 3 bis 15 Vol.-% schwache Säure und 0 und 15 Vol.-% sekundären Alkohol umfasst.

6. Verfahren nach einem der Ansprüche 1 bis 5, **dadurch gekennzeichnet, dass** das Teil in das zweite Bad eingetaucht wird, an die Anode angeschlossen wird und von einer Kathode umgeben ist.

7. Verfahren nach Anspruch 6, **dadurch gekennzeichnet, dass** eine Kathode verwendet wird, die eine Form aufweist, welche an diejenige des Teils angepasst ist, um mit diesem einen im Wesentlichen gleichförmigen Zwischenraum auszubilden.

8. Verfahren nach einem der Ansprüche 2 und 7, **dadurch gekennzeichnet, dass** eine von Löchern durchbohrte Kathode verwendet wird.

9. Verfahren nach einem der Ansprüche 1 bis 8, **dadurch gekennzeichnet, dass** der Schritt des elektrochemischen Ätzens über einen Zeitraum zwischen 1 Min. und 5 Min. durchgeführt wird.

10. Verfahren nach einem der Ansprüche 1 bis 9, **dadurch gekennzeichnet, dass** der Schritt des makrographischen chemischen Ätzens durch Eintauchen in ein erstes Bad, das Chloridionen und Eisen(III)-Chlorid umfasst, vollzogen wird.

11. Verfahren nach einem der Ansprüche 1 bis 10, für die Sichtprüfung von Schaufeln von Turbostrahltriebwerken aus Rhenium und/oder Ruthenium enthaltender monokristalliner Superlegierung.

## Claims

1. Method of revealing structure for the visual inspection of a component made of a single-crystal superalloy containing rhenium and/or ruthenium, the method comprising a chemical macrographic etching phase by means of a first acid bath, **characterized in that**, after the first step, a subsequent step of cleaning the surface of the component is carried out by electrochemical etching by means of a second bath different from the first containing phosphoric acid and at least one weak acid.

2. Method according to Claim 1, **characterized in that** the weak acid is chosen from acetic acid, tartaric acid and citric acid.

3. Method according to any one of Claims 1 and 2, **characterized in that** a second bath also containing a secondary alcohol is used.

4. Method according to Claim 3, **characterized in that** the secondary alcohol is glycol.

5. Method according to any one of Claims 1 to 4, **characterized in that** a second bath containing, by volume, 30 to 80% phosphoric acid, 3 to 15% weak acid and 0 to 15% secondary alcohol is used.

6. Method according to any one of Claims 1 to 5, **characterized in that** the component is immersed in the second bath, connected up as anode and surrounded by a cathode.

7. Method according to Claim 6, **characterized in that** a cathode is used which has a shape tailored to that of the component so as to leave between them an approximately uniform gap.

8. Method according to any one of Claims 2 and 7, **characterized in that** a cathode drilled with holes is used.

9. Method according to any one of Claims 1 to 8, **characterized in that** the electrochemical etching step is carried out for a time of between 1 min and 5 min.

10. Method according to any one of Claims 1 to 9, **characterized in that** the chemical macrographic etching step is carried out by immersing the component in a first bath containing chloride ions and iron perchloride.

11. Method according to any one of Claims 1 to 10 for the visual inspection of turbojet blades made of a single-crystal superalloy containing rhenium and/or ruthenium.
